# NOUVEAU FASCICULE DE BREVET EUROPEEN

(11) **EP 0 852 488 B2**
(45) Date de publication et mention de la décision concernant l'opposition: **20.04.2005**
(45) Mention de la délivrance du brevet: 16.08.2001
(21) Numéro de dépôt: 96931853.4
(22) Date de dépôt: 16.09.1996
(51) Int. Cl.: A61K 7/48, A61K 7/06

(54) **COMPOSITION TOPIQUE CONTENANT UN POLYMERE SILICONE GREFFE ET UNE SILICONE AMINEE ET/OU UNE GOMME OU UNE RESINE DE SILICONE**
EIN GEPFROPFTES SILIKONPOLYMER UND EIN AMINIERTES SILIKON UND/ODER EIN GUMMI ODER SILIKONHARZ ENTHALTENDE TOPISCHE ZUSAMMENSETZUNG
TOPICAL COMPOSITION CONTAINING A SILICONE-GRAFTED POLYMER AND AN AMINE SILICONE AND/OR A SILICONE GUM OR RESIN

(30) Priorité: 29.09.1995 FR 9511481
(43) Date de publication de la demande: 15.07.1998
(73) Titulaire: L'OREAL, 75008 Paris (FR)
(72) Inventeur: DUBIEF, Claude, F-78150 Le Chesnay (FR); DUPUIS, Christine, F-75018 Paris (FR); CAUWET-MARTIN, Danièle, F-75011 Paris (FR)
(74) Mandataire: Dossmann, Gérard
(86) Numéro de dépôt international: PCT/FR1996/001435
(87) Numéro de publication internationale: WO 1997/012594

(56) Documents cités:
- EP-A- 0 017 122
- EP-A- 0 164 668
- EP-A- 0 282 720
- EP-A- 0 412 704
- EP-A- 0 412 707
- EP-A- 0 501 791
- EP-A- 0 530 974
- EP-A- 0 582 152
- EP-A- 0 640 643
- WO-A-89/04161
- WO-A-92/00303
- WO-A-93/23009
- WO-A-93/23446
- WO-A-95/03776
- WO-A-95/05800
- WO-A-97/01321
- FR-A- 2 535 730
- PATENT ABSTRACTS OF JAPAN vol. 11, no. 192 (C-429) [2639] , 19 Juin 1987 & JP,A,62 012712 (NIKKO KEMIKARUZU K.K.), 21 Janvier 1987,
- CHEMICAL ABSTRACTS, vol. 94, no. 6, 9 Février 1981 Columbus, Ohio, US; abstract no. 36111s, page 357; colonne l; XP002001179 & JP,A,08 066 506 (LION CRP.)
- KOSMETIKA,AEROSOLE,RIECHSTOFFE, vol. 114, no. 2, 4 Février 1988, AUGSBURG, pages 51-54, XP002001178 JOSEF ROIDL: "Bedeutung der Silicone in der modernen Kosmetik"
- Produktinformation über Dow Corning 929; November 1986
- Produktinformation über Dow Corning 939 Cationic Emulsion; 1992
- Information About Cosmetic Ingredients, Dow Corning Q2-1401 Fluid; 1987
- Produktinformation ABIL-Quat 3270, 3272, Th.Goldschmidt AG, Juli 1989

## Description

La présente invention a trait à une composition cosmétique ou dermatologique pour le traitement des matières kératiniques, en particulier des cheveux comprenant au moins un polymère siliconé greffé, à squelette polysiloxanique greffé par des monomères organiques non-siliconés et au moins une silicone choisie parmi les silicones comportant au moins une fonction amine non quaternisé les résines de silicone et les gommes de silicone.

On connaît également dans l'état de la technique des polymères à squelette palysiloxanique greffé par des monomères organiques non-siliconés sont choisis préférentiellement parmi ceux décrits dans les demandes EP-A-0582152 et WO 93/23009. Ils sont utilisés en particulier dans les compositions capiliaires pour leur propriétés coiffantes.

On connaît également dans la demande de brevet WO89/04161 des compositions pour le soin des cheveux à base de silicones comportant des groupes aminés en dispersion associées à des de silicones comportant des groupes esters et/ou acides carboxyliques facilement éliminables au shampooings, formant un film après séchage rapide, résistant à l'humidité et à l'eau et à effet prolongé de maintien et de mise en forme.

La demanderesse a découvert de façon surprenante qu'en associant au moins un polymère siliconé à sque-lette polysiloxanique greffé par des monomères organiques non-siliconés avec au moins une gomme et/ou une résine de silicone et/ou une silicone comportant au moins une fonction amine non quaternisée, on obtenait une amélioration du volume, du gonflant de la coiffure tout en ayant de meilleures propriétés coiffantes et/ou de maintien telles que le pouvoir fixant. Les cheveux sont plus "légers" et se coiffent facilement.
Les propriétés de douceur et de toucher des cheveux sont également améliorées.

Par pouvoir fixant de la composition on désignera l'aptitude de cette dernière à donner aux cheveux une cohésion telle que la mise en forme initiale de la coiffure est conservée.

La composition selon l'invention est donc essentiellement caractérisée par le fait qu'elle comprend dans un milieu cosmétiquement ou dermatologiquement acceptable au moins un polymère siliconé greffé, à squelette polysiloxanique greffé par des monomères organiques non-siliconés et au moins une silicone choisie parmi les silicones comportant au moins une fonction amine non quaternisée, les résines de silicone et les gommes de silicone.

Dans ce qui suit, on entend désigner par polymère slliconé, en conformité avec l'acception générale, tous les polymères ou oligomères organosiliciés à structure linéaire ou cyclique, ramifiée ou réticulée, de poids moléculaire variable, obtenus par polymérisation et/ou polycondensation de silanes convenablement fonctionnalisés, et constitués pour l'essentiel par une répétition de motifs principaux dans lesquels les atomes de silicium sont reliés entre eux par des atomes d'oxygène (liaison siloxane ≡Si-O-Si≡), des radicaux hydrocarbonés éventuellement substitués étant directement liés par l'intermédiaire d'un atome de carbone sur lesdits atomes de silicium. Les radicaux hydrocarbonés les plus courants sont les radicaux alkyles notamment en C₁C₁₀ et en particulier méthyle, les radicaux fluoroalkyles, les radicaux aryles et en particulier phényle, et les radicaux alcényles et en particulier vinyle; d'autres types de radicaux susceptibles d'être liés soit directement, soit par l'intermédiaire d'un radical hydrocarboné, à la chaîne siloxanique sont notamment l'hydrogène, les halogènes et en particulier le chlore, le brome ou le fluor, les thiols, les radicaux alcoxy, les radicaux polyoxyalkylènes (ou polyéthers) et en particulier polyoxyéthylène et/ou poly oxypropylène, les radicaux hydroxyles ou hydroxyalkyles, les groupements aminés substitués ou non, les groupements amides, les radicaux acyloxy ou acyloxyalkyles, les radicaux hydroxyalkylamino ou aminoalkyles, des groupements ammonium quaternaires, des groupements amphotères ou bétaïniques, des groupements anioniques tels que carboxylates, thioglycolates, sulfosuccinates, thiosulfates, phosphates et sulfates, cette liste n'étant bien entendu nullement limitative (silicones dites "organomodifiées").

Selon la présente invention, le ou les polymères greffés siliconés qui doivent être utilisés sont ceux qui comprennent une chaîne principale de silicone (ou polysiloxane (≡Si-O-)ₙ) sur laquelle se trouve greffé, à l'intérieur de ladite chaîne ainsi qu'éventuellement à l'une au moins de ses extrémités, au moins un groupement organique ne comportant pas de silicone.

Ces polymères siliconés peuvent être des produits commerciaux existants, ou encore être obtenus selon tout moyen connu de l'homme de l'art, en particulier par réaction entre (i) une silicone de départ correctement fonctionnalistée sur un ou plusieurs de ces atomes de silicium et (li) un composé organique non-siliconé lui-même correctement fonctionnalisé par une fonction qui est capable de venir réagir avec le ou les groupements fonctionnels portés par ladite silicone en formant une liaison covalente ; un exemple classique d'une telle réaction est la réaction d'hydrosylilation entre des groupements ≡Si-H et des groupements vinyliques CH₂=CH-, ou encore la réaction entre des groupements thio-fonctionnels -SH avec ces mêmes groupements vinyliques.

Des exemples de polymères siliconés convenant à la mise en oeuvre de la présente invention, ainsi que leur mode particulier de préparation, sont notamment décrits dans les demandes de brevets EP-A- 0 582 152, WO 93/23009 et WO 95/03776.

Selon un mode particulièrement préféré de réalisation de la présente invention, le polymère siliconé mis en oeuvre comprend le résultat de la copolymérisation radicalaire entre d'une part au moins un monomère organique anionique non-slliconé présentant une insaturation éthylénique et/ou un monomère organique hydrophobe non-siliconé présentant une insaturation éthylénique et d'autre part une silicone présentant dans sa chaîne au moins un groupement fonctionnel capable de venir réagir sur lesdites insaturations éthyléniques desdits monomères non-siliconés en formant une liaison covalente, en particulier des groupements thio-fonctionnels.

Selon la présente invention, lesdits monomères anioniques à insaturation éthylénique sont de préférence choisis, seuls ou en mélange, parmi les acides carboxyliques insaturés, linéaires ou ramifiés, éventuellement partiellement ou totalement neutralisés sous la forme d'un sel, ce ou ces acides carboxyliques insaturés pouvant être plus particulièrement l'acide acrylique, l'acide méthacrylique, l'acide maléique, l'anhydride maléique, l'acide itaconique, l'acide fumarique et l'acide crotonique. Les sels convenables sont notamment les sels d'alcalins, d'alcalino-terreux et d'ammonium. On notera que, de même, dans le polymère siliconé greffé final, le groupement organique à caractère anionique qui comprend le résultat de l'(homo) polymérisation radicalaire d'au moins un monomère anionique de type acide carboxylique insaturé peut être, après réaction, post-neutralisé avec une base (soude, ammoniaque,...) pour l'amener sous la forme d'un sel.

Selon la présente invention, les monomères hydrophobes à insaturation éthylénique sont de préférence choisis, seuls ou en mélange, parmi les esters d'acide acrylique alcanols et/ou les esters d'acide méthacrylique d'alcanols. Les alcanols sont de préférence en C₁-C₁₈ et plus particulièrement en C₁-C₁₂. Les monomères préférentiels sont choisis dans le groupe constitué par le (méth)acrytate d'isooctyle, le (méth) acrylate d'isonyle, le 2-éthylhexyl(méth) acrylate, le (méth)acrylate de lauryle, le (méth) acrylate d'isopentyle, le (méth)acrylate de n-butyle, le (méth)acrylate d'isobutyle, le (méth)acrylate de méthyle, le (méth)acrylate de tertio-butyle, le (méth)acrylate de tridécyle, le (méth) acrylate de stéaryle ou leurs mélanges.

Une famille de polymères siliconés greffés particulièrement bien à la mise en oeuvre de la présente invention est constituée par les polymères siliconés comportant dans leur structure le motif de formule (I) suivant : 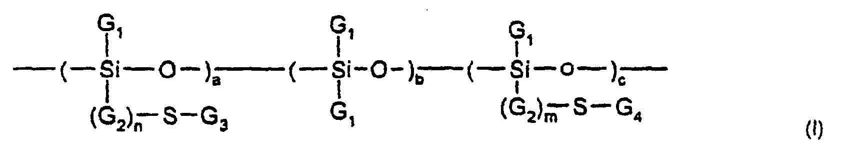 dans lequel les radicaux G₁, identiques ou différents, représentent l'hydrogène ou un radical alkyle en C₁-C₁₀ ou encore un radical phényle ; les radicaux G₂, identiques ou différents, représentent représente un groupe alkylène en C₁-C₁₀ ; G₃ représente un reste polymérique résultant de l'(homo)polymérisation d'au moins un monomère anionique à insaturation éthylénique ; G₄ représente un reste polymérique résultant de l'(homo)polymérisation d'au moins un monomère d'au moins un monomère hydrophobe à insaturation éthylénique ; m et n sont égaux à 0 ou 1 ; a est un nombre entier allant de 0 et 50 ; b est un nombre entier pouvant être compris entre 10 et 350, c est un nombre entier allant de 0 et 50 ; sous réserve que l'un des paramètres a et c soit différent de 0.

De préférence, le motif de formule (I) ci-dessus présente au moins l'une, et encore plus préférentiellement l'ensemble, des caractéristiques suivantes :
- les radicaux G₁ désignent un radical alkyle, de préférence le radical méthyle ;
- n est non nul, et les radicaux G₂ représentent un radical divalent en C₁-C₃, de préférence un radical propylène ;
- G₃ représente un radical polymérique résultant de l'(homo)polymérisation d'au moins un monomère du type acide carboxylique à insaturation éthylénique, de préférence l'acide acrylique et/ou l'acide méthacrylique ;
- G₄ représente un radical polymérique résultant de l'(homo)polymérisation d'au moins un monomère du type (méth) acrylate d'alkyle en C₁-C₁₀, de préférence du type (méth)acrylate d'isobutyle ou de méthyle.

Des exemples de polymères siliconés répondant à la formule (I) sont notamment des polydiméthylsiloxanes (PDMS) sur lesquels sont greffés, par l'intermédiaire d'un chaînon de raccordement de type thiopropylène, des motifs polymères mixtes du type acide poly(méth)acrylique et du type poly(méth)acrylate de méthyle.

D'autres exemples de polymères siliconés répondant à la formule (I) sont notamment des polydiméthylsiloxanes (PDMS) sur lesquels sont greffés, par l'intermédiaire d'un chaînon de raccordement de type thiopropylène, des motifs polymères du type poly (méth)acrylate d'isobutyle.

De préférence, la masse moléculaire en nombre des polymères siliconés de l'invention varie de 10 000 à 1 000 000 environ, et encore plus préférentiellement de 10 000 à 100 000 environ.

Les polymères siliconés greffés conformes à l'invention sont utilisés de préférence en une quantité allant de 0,01 à 20% en poids du poids total de la composition. Plus -préférentiellement, cette quantité varie de 0,1 à 15% en poids et encore plus particulièrement de 0,5 à 10 % en poids.

Parmi les silicones comportant au moins une fonction amine non quaternisée, on peut citer:
(a) les polymères siliconés répondant à la formule (II) suivante :

   R¹ ₐG¹ ₃₋ₐ-Si(OSiG² ₂)ₙ-(OSiG³ _{b}R² _{2-b})ₘ-O-SiG⁴ ₃₋ₐ-R³ ₐ. (II)

   dans laquelle:
   G¹, G², G³ et G⁴, identiques ou différents, désignent un atome d'hydrogène, un groupement phényle, OH, alkyle en C₁-C₁₈, par exemple méthyle, alcényle en C₂-C₁₈, ou alcoxy en C₁-C₁₈ ;
   a, a', identiques ou différents, désignent le nombre 0 ou un nombre entier de 1 à 3, en particulier 0 ;
   b désigne 0 ou 1, et en particulier 1 ;
   m et n sont des nombres tels que la somme (n + m) peut varier notamment de 1 à 2 000 et en particulier de 50 à 150, n pouvant désigner un nombre de 0 à 1 999 et notamment de 49 à 149 et m pouvant désigner un nombre de 1 à 2 000, et notamment de 1 à 10 ;
   R¹, R², R³, R⁴, identiques ou différents, désignent un radical monovalent de formule-C_{q}H_{2q}Oₛ R⁵ₜL dans laquelle q est un nombre de 1 à 8, s et t, identiques ou différents, sont égaux à 0 ou à 1, R⁵ désigne un groupement alkylène éventuellement hydroxylé et L est un groupement aminé choisi parmi les groupements :

      -NR"-CH₂-CH₂-N'(R")₂

      -N(R")₂
   dans lesquels R" peut désigner hydrogène, phényle, benzyle, ou un radical hydrocarboné saturé monovalent, par exemple un radical alkyle ayant de 1 à 20 atomes de carbone.
(b)- les composés de formule (III):

   NH-[(CH₂)₃-Si[OSi(CH₃)₃]]₃ (III)

Ce composé correspond à la dénomination CTFA "amino bis-propyldiméthicone".

Des produits correspondant à la formule (II) sont par exemple les polysiloxanes dénommés dans le dictionnaire CTFA "amodiméthicone" et répondant à la formule (IV) suivante : 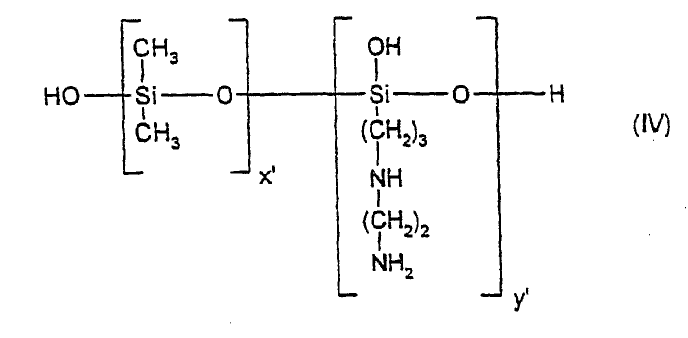 dans laquelle x' et y' sont des nombres entiers dépendant du poids moléculaire, généralement tels que ledit poids moléculaire est compris entre 5 000 et 20 000 environ.

Un produit correspondant à la formule (II) est le polymère dénommé dans le dictionnaire CTFA "triméthylsilylamodiméthicone", répondant à la formule (V) : 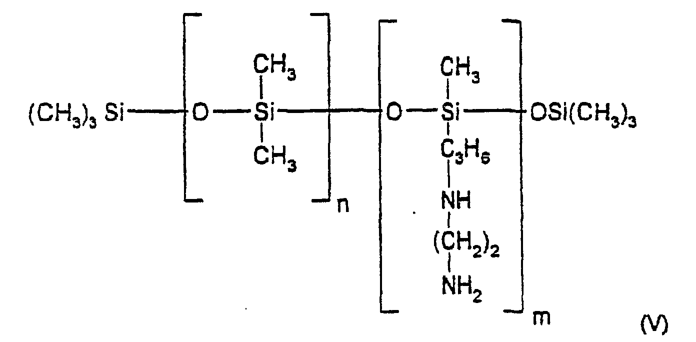 dans laquelle n et m ont les significations données ci-dessus (cf. formule II).

Un produit commercial répondant à cette définition est un mélange (90/10 en poids) d'un polydiméthyisiloxane à groupements aminoéthyl aminoisobutyle et d'un polydiméthylsiloxane vendu sous la dénomination Q2-8220 par la société DOW CORNING.

De tels polymères sont décrits par exemple dans la demande de brevet EP-A-95238.

Un polymère répondant à la formule (III) est le polymère vendu par la Société Union Carbide sous la dénomination "Ucar Silicone ALE 56.

Lorsque ces polymères siliconés sont mis en oeuvre, une forme de réalisation particulièrement Intéressante est leur utilisation conjointe avec des agents de surface cationiques et/ou non ioniques. On peut utiliser par exemple le produit vendu sous la dénomination "Emulsion Cationique DC 929" par la Société DOW CORNING qui comprend, outre l'amodiméthicone, un agent de surface cationique comprenant un mélange de produits répondant à la formule : 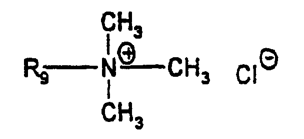 dans lequel R₉ désigne des radicaux alcényle et/ou alcoyle ayant de 14 à 22 atomes de carbone, dérivés des acides gras du suif,
en association avec un agent de surface non ionique de formule :
C₉H₁₉-C₆H₄-(OC₂H₄)₁₀-OH ,connu sous la dénomination CTFA "Nonoxynol 10".

Un autre produit commercial utilisable selon l'invention est le produit vendu sous la dénomination "Dow Corning Q2 7224" par la Société Dow Coming comportant en association le triméthylsilylamodiméthicone de formule (V), un agent de surface non ionique de formule : C₈H₁₇-C₆H₄-(OCH₂CH₂)ₙ-OH où n = 40 dénommé encore octoxynol-40, un autre agent de surface non ionique de formule :
C₁₂H₂₅-(OCH₂-CH₂)ₙ-OH où n = 6 encore dénommé isolaureth-6, et du glycol.

Les gommes de silicone, conformes à la présente invention, sont des polydiorganosiloxanes de fortes masses moléculaires, comprises entre 200.000 et 2 000 000, utilisées seules ou en mélange dans un solvant choisi parmi les silicones volatiles, les huiles polydiméthylsiloxane, les huiles polyméthylphénylsiloxane ou polydiphényldiméthylsiloxane, les isoparaffines, le chlorure de méthylène, le pentane, les hydrocarbures ou leurs mélanges.

On utilise de préférence une gomme de silicone ayant un poids moléculaire inférieur à 1 500 000. Les gommes de silicone sont par exemple un polydiméthylsiloxane, un polyphénylméthylsiloxane, un poly(diphénylsiloxane diméthylsiloxane), un poly(diméthylsiloxane méthylvinylsiloxane), un poly-(diméthylsiloxane phénylméthyl siloxane), un poly-(diphénylsiloxane diméthylsiloxane méthylvinylsiloxane). Ces gommes de silicone peuvent être terminées en bout de chaîne par des groupements triméthylsilyl ou diméthylhydroxysilyl.

En particulier, on peut utiliser une gomme de silicone répondant à la formule (VI) : 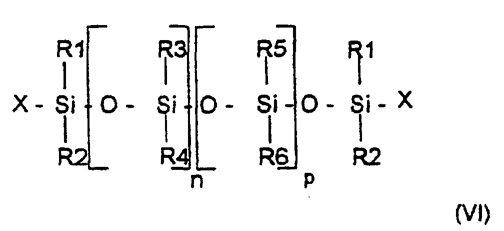 dans laquelle :
R₁, R₂, R₅ et R₆ sont, ensemble ou séparément, un radical alkyle ayant 1 à 6 atomes de carbone,
R₃ et R₄ sont, ensemble ou séparément, un radical alkyle ayant de 1 à 6 atomes de carbone, ou un radical aryle,
X est un radical alkyle ayant de 1 à 6 atomes de carbone, un radical hydroxyle ou un radical vinyle,
n et p étant choisis de manière à conférer à la gomme de silicone une viscosité supérieure à 100 000 mPa.s, de préférence supérieure à 500 000 mPa.s.

De manière générale, n et p peuvent prendre des valeurs de 0 à 5000, de préférence de 0 à 3000.
La gomme de silicone peut être introduite dans la composition telle quelle ou sous forme diluée dans une huile de silicone telle qu'un PDMS (polydiméthylsiloxane) volatile ou non volatile.
Comme gomme de silicone utilisable selon l'invention, on peut citer celles pour lesquelles :
. les substituants R₁ à R₆ et X représentent un groupement méthyle, p = 0 et n = 2700, comme celle vendue sous la dénomination SE30 par la société Général Electric,
. les substituants R₁ à R₆ et X représentent un groupement méthyle, p = 0 et n = 2300, comme celle vendue sous la dénomination AK 500000 par la société Waker, .
. les substituants R₁ à R₆ représentent un groupement méthyle, le substituant X représente un groupement hydroxyle, p = 0 et n = 2700, en solution à 13 % dans du cyclopentasiloxane, comme celle vendue sous la dénomination Q2-1401 par la société DOW CORNING,
. les substituants R₁ à R₆ représentent un groupement méthyle, le substituant X représente un groupement hydroxyle, p = 0 et n = 2700, en solution à 13% dans le diméthicone, comme celle vendue sous la dénomination Q2-1403 par la société DOW CORNING,
. les substituants R₁, R₂ R₅, R₆ et X représentent un groupement méthyle, les substituants R₃ et R₄ représentent un groupement aryle tel que le poids moléculaire du composé soit de 600 000, comme celle vendue sous la dénomination 761 par la société RHONE-POULENC.

Les résines de silicone utilisables conformément à l'invention, sont des systèmes siloxaniques réticulés renfermant les unités : R₂SiO_{2/2}, RSiO_{3/2}, et SiO_{4/2} dans lesquelles R représente un groupement hydrocarboné possédant de 1 à 6 atomes de carbone ou un groupement phényle. Parmi ces produits, ceux particulièrement préférés sont ceux dans lesquels R désigne un radical alkyle inférieur (C₁-C₆) ou un radical phényle.

Parmi ces résines, on peut citer le produit vendu sous la dénomination "DOW CORNING 593" ou ceux vendus sous les dénominations "SILICONE FLUID SS 4230" et "SS 4267" par la société GENERAL ELECTRIC et qui sont des "diméthyl/ triméthylpolysiloxane".

Les gommes ou les résines de silicone et/ou les silicones aminées sont utilisées de préférence en une quantité comprise entre 0,01 et 50% en poids du poids total de la composition, de préférence entre 0,05 et 20% en poids. Encore plus préférentiellement, cette quantité est comprise entre 0,1 et 10% en poids.

Le milieu cosmétiquement ou dermatologiquement acceptable est de préférence constitué par de l'eau ou un mélange d'eau et de solvants cosmétiquement acceptables tels que des monoalcools, des polyalcools, des éthers de glycol ou des esters d'acides gras, qui peuvent être utilisés seuls ou en mélange.

On peut citer plus particulièrement les alcools inférieurs tels que l'éthanol, l'isopropanol, les polyalcools tels que le diéthylèneglycol, les éthers de glycol, les alkyléthers de glycol ou de diéthylèneglycol.

Les polymères siliconés greffés selon l'invention peuvent être dissous dans ledit milieu cosmétiquement acceptable ou utilisés sous forme de dispersion aqueuse de particules.

La composition de l'invention peut également contenir au moins un additif choisi parmi les épaississants, les esters d'acides gras, les esters d'acides gras et de glycérol, les silicones volatiles, les tensioactifs, les parfums, les conservateurs, les filtres solaires, les protéines, les vitamines, les polymères, les huiles végétales, animales, minérales ou synthétiques et tout autre additif classiquement utilisé dans le domaine cosmétique.

Ces additifs sont présents dans la composition selon l'invention dans des proportions pouvant aller de 0 à 20% en poids par rapport au poids total de la composition. La quantité précise de chaque additif est fonction de sa nature et est déterminée facilement par l'homme de l'art.

Bien entendu, l'homme de l'art veillera à choisir le ou les éventuels composés à ajouter à la composition selon l'invention de manière telle que les propriétés avantageuses attachées intrinsèquement à la composition conforme à l'invention ne soient pas, ou substantiellement pas, altérées par l'addition envisagée.

Les compositions selon l'invention peuvent se présenter sous forme de gel, de lait, de crème, de lotion plus ou moins épaissie ou de mousse.

Les compositions selon l'invention sont utilisées comme produits rincés ou comme produits non-rincés notamment pour le lavage, le soin, le conditionnement, le maintien de la coiffure ou la mise en forme des matières kératiniques telles que les cheveux.

Elles sont plus particulièrement des produits de coiffage tels que des lotions de mise en plis, des lotions pour le brushing, des compositions de fixation (laques) et de coiffage. Les lotions peuvent être conditionnées sous diverses formes notamment dans des vaporisateurs, des flacons pompes ou dans des récipients aérosols afin d'assurer une application de la composition sous forme vaporisée ou sous forme de mousse. De telles formes de conditionnement sont indiquées, par exemple, lorsqu'on souhaite obtenir un spray, une laque ou une mousse pour la fixation ou le traitement des cheveux.

Les compositions peuvent être également des shampooings, des compositions à rincer ou non, à appliquer avant ou après un shampooing, une coloration, une décoloration, une permanente ou un défrisage.

Lorsque la composition selon l'invention est conditionnée sous forme d'aérosol en vue d'obtenir une laque ou une mousse aérosol, elle comprend au moins un agent propulseur qui peut être choisi parmi les hydrocarbures volatils tels que le n-butane, le propane, l'isobutane, le pentane, un hydrocarbure chloré et/ou fluoré et leurs mélanges. On peut également utiliser en tant qu'agent propulseur le gaz carbonique, le protoxyde d'azote, le diméthyléther, l'azote, l'air comprimé et leurs mélanges.

L'invention a encore pour objet un procédé de traitement des matières kératiniques telles que les cheveux consistant à appliquer sur ceux-ci une composition telle que définie précédemment puis à effectuer éventuellement un rinçage à l'eau.

L'invention va être maintenant plus complètement illustrée à l'aide des exemples suivants qui ne sauraient être considérés comme la limitant aux modes de réalisation décrits. Dans tout ce qui suit, MA signifie Matière Active.

### EXEMPLE 1

On a préparé un shampooing de composition suivante:
- Lauryl (C₁₂/C₁₄ 70/30) éther sulfate de sodium oxyéthyléné à 2,2 moles d'oxyde d'éthylène en solution aqueuse à 28 % de MA vendu sous le nom d'Empicol ESB 31/F par la société Albright et Wilson 14 g MA
- Cocoylbétaïne en solution aqueuse à 28 % de MA 3 g MA
- Polymère slliconé greffé de formule (I) de structure polyméthyl/méthylsiloxane à groupements propyl thio-3 polyméthacrylate d'isobutyle en solution dans une silicone volatile cyclique 1 g
- Mélange (47/53 en poids) de 1-(hexadécyloxy)-2-octadecanol et d'alcoolcétylique 2 g
- Monoisopropanolamide d'acide de coprah 2 g
- Polydiméthylsiloxane à groupements aminoéthyl aminopropyl en émulsion cationique à 35 % dans l'eau 1 g MA vendu par Dow Corning sous le nom de DC 939
- Parfum, séquestrant, conservateur
- Eau q.s.p. 100 g

Le pH est ajusté à 5 par l'addition de soude.

### EXEMPLE 2

On a préparé un après-shampooing à rincer de composition suivante:
- Polymère siliconé greffé de formule (I) de structure polyméthyl/méthylsiloxane à groupements propyl thio-3 polyméthacrylate d'isobutyle en solution dans une silicone volatile cyclique 2 g
- Copolymère réticulé de chorure de méthacrylate de triméthyl éthyl ammonium et d'acrylamide (42/58 en poids) vendu en dispersion dans l'huile à 50 % de MA par la société Allied Colloïds sous le nom de Salcare SC 92 1 gMA
- Mélange (56/44 en poids) d'un polydiméthylsiloxane α,Ω-dihydroxy et de silicones volatiles cycliques vendu sous la dénomination Q2-1401 par la société Dow Corning 2 g
- Parfum, conservateur
- Eau q.s.p. 100 g

Le pH est ajusté à 7 par l'addition de soude.

## Revendications

1. Composition cosmétique ou dermatologique destinée au traitement des matières kératiniques, **caractérisée par le fait qu'**elle comprend dans un milieu cosmétiquement ou dermatologiquement acceptable au moins :
(a) un polymère siliconé à squelette polysiloxane greffé par un groupement organique ne comportant pas de silicone susceptible d'être obtenu par copolymérisation radicalaire entre d'une part (I) au moins un monomère organique anionique non-siliconé présentant une insaturation éthylénique et/ou un monomère organique hydrophobe non-siliconé présentant une insaturation éthylénique et d'autre part (II) un polysiloxane présentant dans sa chaîne au moins un, et de préférence plusieurs, groupements fonctionnels capables de venir réagir sur lesdites insaturations éthyléniques desdits monomères non-siliconés ; et
(b) au moins une silicone choisie parmi les silicones comportant au moins une fonction amine non quaternisée les résines de silicone et les gommes de silicone.

2. Composition selon la revendication 1, **caractérisée par le fait que** le monomère organique anionique à insaturation éthylénique est choisi, seul ou sous forme de mélange de monomères, parmi les acides carboxyliques insaturés, linéaires ou ramifiés.

3. Composition selon la revendication 2, **caractérisée par le fait que** le monomère organique anionique à insaturation éthylénique est choisi, seul ou sous forme de mélange de monomères, parmi l'acide acrylique, l'acide méthacrylique, l'acide maléique, l'anhydride maléique, l'acide itaconlque, l'acide fumarique et l'acide crotonique ou leurs sels d'alcalins, d'alcalino-terreux ou d'ammonium, ou leurs mélanges.

4. Composition selon la revendication 1, **caractérisée par le fait que** le monomère organique hydrophobe à insaturation éthylénique est choisi, seul ou en mélange de monomères, parmi les esters d'acide acrylique d'alcanol et/ou les esters d'acide méthacrylique d'alcanol, de préférence l'alcanol étant en C₁-C₁₈.

5. Composition selon la revendications 4, **caractérisée par le fait que** le monomère organique hydrophobe à insaturation éthylénique est choisi, seul ou en mélange de monomères dans le groupe constitué par le (méth)acrylate d'isooctyle, le (méth) acrylate d'isonyle, le 2-éthylhexyl(méth)acrylate, le (méth)acrylate de lauryle, le (méth) acrylate d'isopentyle, le (méth)acrylate de n-butyle, le (méth)acrylate d'isobutyle, le (méth)acrylate de méthyle, le (méth) acrylate de tertio-butyle, le (méth)acrylate de tridécyle, le (méth)acrylate de stéaryle.

6. Composition selon l'une quelconque des revendications 1 à 5, **caractérisée par le fait que** le polymère siliconé greffé comprend sur la chaîne silicone principale, au moins un groupement organique à caractère anionique obtenu par l'(homo) polymérisation radicalaire d'au moins un monomère anionique de type acide carboxylique insaturé partiellement ou totalement neutralisé sous la forme d'un sel.

7. Composition selon l'une quelconque des revendications 1 à 6, **caractérisée par le fait que** le polymère siliconé greffé est choisi parmi les polymères siliconés comportant dans leur structure le motif de formule (I) suivant : 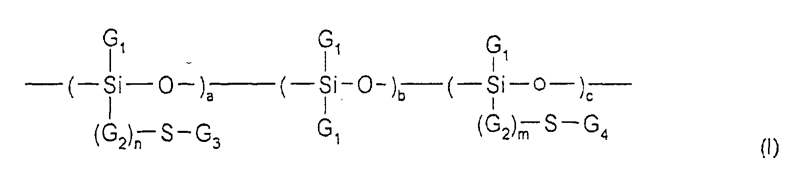 dans lequel les radicaux G₁, identiques ou différents, représentent l'hydrogène ou un radical alkyle en C₁-C₁₀ ou encore un radical phényle ; les radicaux G₂, identiques ou différents, représentent un groupe alkylène en C₁-C₁₀ ; G₃ représente un reste polymérique résultant de l'(homo)polymérisation d'au moins un monomère anionique à insaturation éthylénique ; G₄ représente un reste polymérique résultant de l'(homo) polymérisation d'au moins un monomère d'au moins un monomère hydrophobe à insaturation éthylénique ; m et n sont égaux à 0 ou 1 ; a est un nombre entier allant de 0 et 50 ; b est un nombre entier pouvant être compris entre 10 et 350, c est un nombre entier allant de 0 et 50 ; sous réserve que l'un des paramètres a et c soit différent de 0.

8. Composition selon la revendication 7, **caractérisée par le fait que** le motif de formule (I) présente au moins l'une des caractéristiques suivantes :
- les radicaux G₁ désignent un radical alkyle en C₁-C₁₀;
- n est non nul, et les radicaux G₂ représentent un radical divalent en C₁-C₃ ;
- G₃ représente un radical polymérique résultant de l'(homo)polymérisation d'au moins un monomère du type acide carboxylique à insaturation éthylénique ;
- G₄ représente un radical polymérique résultant de l'(homo)polymérisation d'au moins un monomère du type type (méth)acrylate d'alkyle en C₁-C₁₀.

9. Composition selon la revendication 7 ou 8, **caractérisée par le fait que** le motif de formule (I) présente simultanément les caractéristiques suivantes :
- les radicaux G₁ désignent un radical méthyle ;
- n est non nul, et les radicaux G₂ représentent un radical propylène ;
- G₃ représente un radical polymérique résultant de l'(homo)polymérisation d'au moins l'acide acrylique et/ou l'acide méthacrylique ;
- G₄ représente un radical polymérique résultant de l'(homo)polymérisation d'au moins le (méth)acrylate d'iso-butyle ou de méthyle.

10. Composition selon l'une quelconque des revendications 1 à 9, **caractérisée par le fait que** la masse moléculaire en nombre du polymère siliconé greffé varie de 10 000 à 1 000 000, et encore plus préférentiellement de 10 000 à 100 000.

11. Composition selon l'une quelconque des revendications 1 à 10, **caractérisé par le fait que** le ou les polymères greffés silionés sont présents dans des concentrations allant de 0,01 à 20% en poids par rapport au poids total de la composition,de préférence de 0,1 à 15% en poids et plus particulièrement de 0,5 à 10 % en poids.

12. Composition selon l'une quelconque des revendications 1 à 11, **caractérisée par le fait que** les silicones comportant au moins une fonction amine non quaternisée, les résines et les gommes de silicone sont utilisées en une quantité comprise entre 0,01 et 50% en poids du poids total de la composition, de préférence entre 0,05 et 20% en poids, plus préférentiellement, cette quantité est comprise entre 0,1 et 10% en poids.

13. Composition selon l'une quelconque des revendications précédentes, **caractérisée par le fait que** la silicone comportant au moins une fonction amine est choisie parmi :
(a) les polymères siliconés répondant à la formule (II) suivante
R¹ ₐG¹ ₃₋ₐ-Si(OSiG² ₂)ₙ-(OSiG³ _{b}R² _{2-b})ₘ-O-SiG⁴ _{3-a,}-R³ _{a,} (II)
dans laquelle :
G¹, G², G³ et G⁴ identiques ou différents, désignent un atome d'hydrogène, un groupement phényle, OH, alkyle en C₁-C₁₈, par exemple méthyle, alcényle en C₂-C₁₈, ou alcoxy en C₁-C₁₈
a, a', identiques ou différents, désignent le nombre 0 ou un nombre entier de 1 à 3, en particulier 0,
b désigne 0 ou 1, et en particulier 1,
m et n sont des nombres tels que la somme (n + m) peut varier notamment de 1 à 2 000 et en particulier de 50 à 150, n pouvant désigner un nombre de 0 à 1 999 et notamment de 49 à 149 et m pouvant désigner un nombre de 1 à 2 000, et notamment de 1 à 10 ;
R¹, R², R³, R⁴, identiques ou différents, désignent un radical monovalent de formule - C_{q}H_{2q}Oₛ R⁵ₜL dans laquelle q est un nombre de 1 à 8, s et t, identiques ou différents, sont égaux à 0 ou à 1, R⁵ désigne un groupement alkylène éventuellement hydroxylé et L est un groupement aminé choisi parmi les groupements :
- NR"-CH₂-CH₂-N'(R")₂
- N(R")₂
dans lesquels R" peut désigner hydrogène, phényle, benzyle, ou un radical hydrocarboné saturé monovalent, par exemple un radical alkyle ayant de 1 à 20 atomes de carbone.
(b)- les composés de formule (III) suivante :
NH-[(CH₂)₃-Si[OSi(CH₃)₃]]₃ (III)

14. Composition selon la revendication 13, **caractérisée par le fait que** la silicone comportant au moins une fonction amine de formule (II) est choisie parmi :
(a) les polysiloxanes dénommés dans le dictionnaire CTFA "amodiméthicone" et répondant à la formule (IV) suivante : 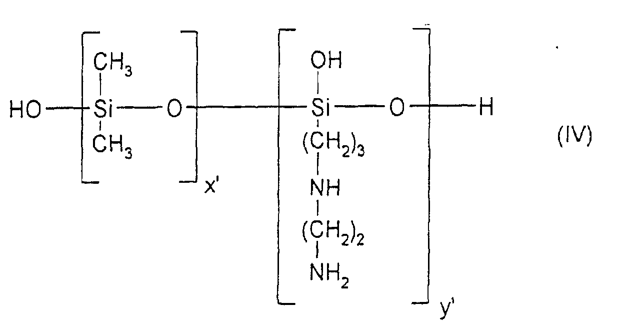 dans laquelle x' et y' sont des nombres entiers dépendant du poids moléculaire, généralement tels que ledit poids moléculaire est compris entre 5 000 et 20 000;
(b) les polymères dénommés dans le dictionnaire CTFA "triméthylsilyl amodiméthicone", répondant à la formule (V) : 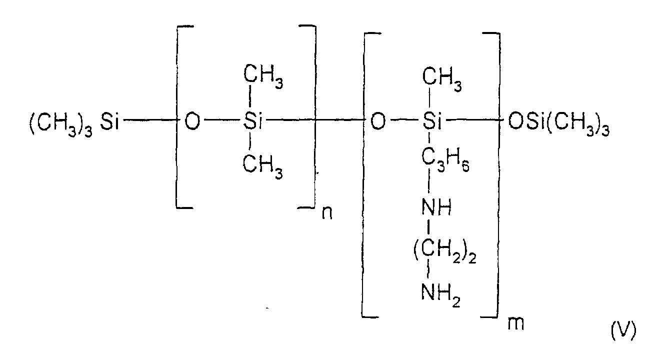
dans laquelle n et m ont les significations données à la revendication précédente ;

15. Composition selon l'une quelconque des revendications 1 à 14, **caractérisée par le fait que** les gommes de silicone sont des polydiorganosiloxane de fortes masses moléculaires, comprises entre 200.000 et 2 000 000.

16. Composition selon l'une quelconque des revendications 1 à 15, **caractérisée par le fait que** les polydiorganosiloxanes sont un polydiméthylsiloxane, un polyphénylméthylsiloxane, un poly(diphénylsiloxane diméthylsiloxane), un poly (diméthylsiloxane méthylvinylsiloxane), un poly(diméthylsiloxane phénylméthyl-siloxane), un poly(diphénylsiloxane diméthylsiloxane méthylvinylsiloxane).

17. Composition selon l'une quelconques des revendications 15 et 16, **caractérisée par le fait que** la gomme de silicone répond à la formule (VI) : 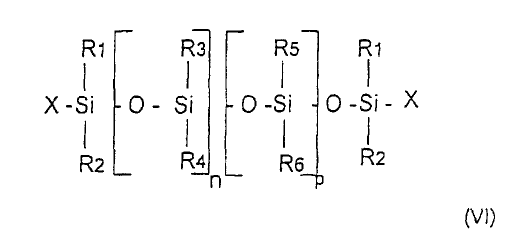 dans laquelle :
R₁, R₂, R₅ et R₆ sont, ensemble ou séparément, un radical alkyle ayant 1 à 6 atomes de carbone,
R₃ et R₄ sont, ensemble ou séparément, un radical alkyle ayant de 1 à 6 atomes de carbone, ou un radical aryle,
X est un radical alkyle ayant de 1 à 6 atomes de carbone, un radical hydroxyle ou un radical vinyle,
n et p étant choisis de manière à conférer à la gomme de silicone une viscosité supérieure à 100 000 mPa. s, de préférence supérieure à 500 000 mPa.s.

18. Composition selon l'une quelconque des revendications 1 à 17, **caractérisée par le fait qu'**elle contient en plus au moins un additif choisi dans le groupe constitué par les épaississants, les esters d'acides gras, les esters d'acides gras et de glycérol, les silicones, les tensioactifs, les parfums, les conservateurs, les filtres solaires, les protéines, les vitamines, les polymères, les huiles végétales, animales, minérales ou synthétiques et tout autre additif classiquement utilisé dans le domaine cosmétique.

19. Composition selon l'une quelconque des revendications 1 à 18, **caractérisée par le fait que** le milieu cosmétiquement ou dermatologiquement acceptable est constitué par de l'eau ou un mélange d'eau et d'au moins un solvant cosmétiquement acceptable.

20. Composition selon la revendication 19, **caractérisée par le fait que** les solvants cosmétiquement acceptables sont choisis dans le groupe constitué par les monoalcools, les polyalcools, les éthers de glycol, les esters d'acides gras et leurs mélanges.

21. Composition selon l'une quelconque des revendications 1 à 20, **caractérisée par le fait que** les matières kératiniques sont des cheveux.

22. Composition selon l'une quelconque des revendications 1 à 21, **caractérisée par le fait qu'**elle se présente sous forme de gel, de lait, de crème, de lotion plus ou moins épaissie ou de mousse.

23. Composition selon l'une quelconque des revendications 1 à 22, **caractérisée par le fait qu'**elle est un produit de coiffage.

24. Composition selon l'une quelconque des revendications 1 à 23, **caractérisée par le fait qu'**elle est un produit capillaire choisi dans le groupe constitué par des shampooings ; des produits capillaires à rincer ou non, à appliquer avant ou après un shampooing, une coloration, une décoloration, une permanente ou un défrisage.

25. Composition selon l'une quelconque des revendications 1 à 24, **caractérisée par le fait qu'**elle est conditionnée sous forme de vaporisateur, de flacon pompe ou bien dans un récipient aérosol en vue d'obtenir un spray, une laque ou une mousse.

26. Composition selon l'une quelconque des revendications 1 à 25, **caractérisée par le fait que** le polymère siliconé greffé est dissous dans le milieu cosmétiquement ou dermatologiquement acceptable ou utilisé sous forme de dispersion aqueuse de particules.

27. Procédé cosmétique de traitement des matières kératiniques en particulier des cheveux, **caractérisé par le fait qu'**il consiste à appliquer sur lesdites matières une composition telle que définie selon l'une quelconque des revendications 1 à 26 puis à effectuer éventuellement un rinçage à l'eau.

## Patentansprüche

1. Kosmetische oder dermatologische Zusammensetzung, die zur Behandlung von Keratinsubstanzen vorgesehen ist, **dadurch gekennzeichnet, dass** sie in einem kosmetisch oder dermatologisch akzeptablen Medium mindestens enthält:
(a) ein Siliconpolymer mit einem Polysiloxangerüst, auf das eine organische Gruppe, die kein Silicon enthält, gepfropft ist, das durch radikalische Copolymerisation (i) mindestens eines nicht siliconhaltigen, anionischen, organischen Monomers, das eine ethylenische Doppelbindung aufweist, und/oder eines nicht siliconhaltigen, hydrophoben, organischen Monomers, das eine ethylenische Doppelbindung aufweist, und (ii) eines Polysiloxans hergestellt werden kann, das in seiner Kette mindestens eine und vorzugsweise mehrere funktionelle Gruppe enthält, die mit den ethylenischen Doppelbindungen der nicht siliconhaltigen Monomere reagieren können; und
(b) mindestens ein Silicon, das unter den Siliconen, die mindestens eine Aminogruppe enthalten, die nicht quaternisiert ist, den Siliconharzen und den Silicongummis ausgewählt ist.

2. Zusammensetzung nach Anspruch 1, **dadurch gekennzeichnet, dass** das anionische, organische Monomer mit ethylenischer Doppelbindung einzeln oder in Form von Monomerengemischen unter den geradkettigen oder verzweigten, ungesättigten Carbonsäuren ausgewählt ist.

3. Zusammensetzung nach Anspruch 2, **dadurch gekennzeichnet, dass** das anionische, organische Monomer mit ethylenischer Doppelbindung einzeln oder in Form von Monomerengemischen unter Acrylsäure, Methacrylsäure, Maleinsäure, Maleinsäureanhydrid, Itaconsäure, Fumarsäure und Crotonsäure oder deren Alkalisalzen, Erdalkalisalzen oder Ammoniumsalzen oder deren Gemischen ausgewählt ist.

4. Zusammensetzung nach Anspruch 1, **dadurch gekennzeichnet, dass** das hydrophobe, organische Monomer mit ethylenischer Doppelbindung einzeln oder in Form von Monomerengemischen unter den Estern von Acrylsäure und Alkanolen und/oder Estern von Methacrylsäure und Alkanolen oder deren Gemischen ausgewählt ist, wobei die Alkanole vorzugsweise 1 bis 18 Kohlenstoffatome aufweisen.

5. Zusammensetzung nach Anspruch 4, **dadurch gekennzeichnet, dass** das hydrophobe, organische Monomer mit ethylenischer Doppelbindung einzeln oder in Form von Monomerengemischen unter Isooctyl(meth)acrylat, Isononyl(meth)acrylat, 2-Ethylhexyl(meth)acrylat, Lauryl(meth)acrylat, Isopentyl(meth)acrylat, *n*-Butyl(meth)acrylat, Isobutyl(meth)acrylat, Methyl(meth)acrylat, *t*-Butyl(meth)acrylat, Tridecyl(meth)acrylat und Stearyl(meth)acrylat ausgewählt ist.

6. Zusammensetzung nach einem der Ansprüche 1 bis 5, **dadurch gekennzeichnet, dass** das gepfropfte Siliconpolymer in der Siliconhauptkette mindestens eine organische Gruppe mit anionischen Eigenschaften enthält, die durch radikalische (Homo)polymerisation mindestens eines anionischen Monomers vom Typ einer ungesättigten, ganz oder teilweise in Form eines Salzes neutralisierten Carbonsäure erhalten wird.

7. Zusammensetzung nach einem der Ansprüche 1 bis 6, **dadurch gekennzeichnet, dass** das gepfropfte Siliconpolymere unter den Siliconpolymeren ausgewählt ist, die in ihrer Struktur die Einheit der folgenden Formel (I) aufweisen: 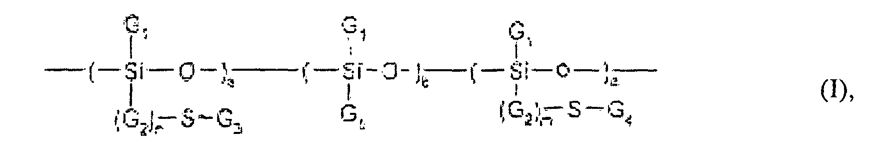 worin die Gruppen G₁, die identisch oder voneinander verschieden sind, Wasserstoff, C₁₋₁₀-Alkyl oder Phenyl; die Gruppen G₂, die identisch oder voneinander verschieden sind, C₁₋₁₀-Alkylen; G₃ eine Polymergruppe, die aus der (Homo)polymerisation mindestens eines anionischen Monomers mit ethylenischer Doppelbindung resultiert; G₄ eine Polymergruppe, die aus der (Homo)-polymerisation mindestens eines hydrophoben Monomers mit ethylenischer Doppelbindung resultiert; m und n 0 oder 1; a 0 oder eine ganze Zahl im Bereich von 1 bis 50; b eine ganze Zahl im Bereich von 10 bis 350; und c 0 oder eine ganze Zahl von 1 bis 50 bedeuten, mit der Maßgabe, dass einer der Parameter a oder c von 0 verschieden ist.

8. Zusammensetzung nach Anspruch 7, **dadurch gekennzeichnet, dass** die Einheit der Formel (I) mindestens eine der folgenden Eigenschaften aufweist:
- die Gruppen G₁ bedeuten C₁₋₁₀-Alkyl;
- n ist nicht Null und die Gruppen G₂ bedeuten eine zweiwertige Gruppe mit 1 bis 3 Kohlenstoffatomen;
- G₃ bedeutet eine Polymergruppe, die aus der (Homo)polymerisation mindestens eines Monomers vom Typ einer Carbonsäure mit ethylenischer Doppelbindung resultiert;
- G₄ bedeutet eine Polymergruppe, die aus der (Homo)polymerisation mindestens eines Monomers vom Typ C₁₋₁₀-Alkyl(meth)acrylat resultiert.

9. Zusammensetzung nach Anspruch 7 oder 8, **dadurch gekennzeichnet, dass** die Einheit der Formel (I) gleichzeitig die folgenden Eigenschaften aufweist:
- die Gruppen G₁ bedeuten Methyl;
- n ist nicht Null und die Gruppen G₂ bedeuten Propylen;
- G₃ bedeutet eine Polymergruppe, die aus der (Homo)polymerisation von zumindest Acrylsäure und/oder Methacrylsäure resultiert;
- G₄ bedeutet eine Polymergruppe, die aus der (Homo)polymerisation von zumindest Isobutyl(meth)acrylat oder Methyl(meth)acrylat resultiert.

10. Zusammensetzung nach einem der Ansprüche 1 bis 9, **dadurch gekennzeichnet, dass** das Zahlenmittel des Molekulargewichts der gepfropften Siliconpolymere im Bereich von 10 000 bis 1 000 000 und noch bevorzugter 10 000 bis 100 000 liegt.

11. Zusammensetzung nach einem der Ansprüche 1 bis 10, **dadurch gekennzeichnet, dass** das gepfropfte Siliconpolymer oder die gepfropften Siliconpolymere in Konzentrationen von 0,01 bis 20 Gew.-%, bezogen auf das Gesamtgewicht der Zusammensetzung, vorzugsweise im Bereich von 0,1 bis 15 Gew.-% und besonders bevorzugt im Bereich von 0,5 bis 10 Gew.-% vorliegen.

12. Zusammensetzung nach einem der Ansprüche 1 bis 11, **dadurch gekennzeichnet, dass** die Silicone, die mindestens eine nicht quaternisierte Aminogruppe enthalten, die Siliconharze und die Silicongummis in einem Mengenanteil von 0,01 bis 50 Gew.-%, vorzugsweise 0,05 bis 20 Gew.-% und noch bevorzugter 0,1 bis 10 Gew.%, bezogen auf das Gesamtgewicht der Zusammensetzung, verwendet werden.

13. Zusammensetzung nach einem der vorhergehenden Ansprüche, **dadurch gekennzeichnet, dass** die Silicone, die mindestens eine Aminogruppe enthalten, ausgewählt sind unter:
(a) Siliconpolymeren, die der folgenden Formel (II) entsprechen:
R¹ ₐG¹ ₃₋ₐ-Si(OSiG² ₂)ₙ-(OSiG³ _{b}R² _{2-b})ₘ-O-SiG⁴ _{3-a'}-R³ _{a'} (II),
worin bedeuten:
G¹, G², G³ und G⁴, die identisch oder voneinander verschieden sind, Wasserstoff, Phenyl, OH, C₁₋₁₈-Alkyl, beispielsweise Methyl, C₂₋₁₈-Alkenyl oder C₁₋₁₈-Alkoxy; a und a', die identisch oder voneinander verschieden sind, 0 oder eine ganze Zahl von 1 bis 3 und insbesondere 0;
b 0 oder 1 und insbesondere 1;
m und n Zahlen, deren Summe (n + m) insbesondere im Bereich von 1 bis 2 000 und besonders 50 bis 150 liegen kann, wobei n eine Zahl von 0 bis 1 999 und insbesondere 49 bis 149 und m eine Zahl von 1 bis 2 000 und insbesondere 1 bis 10 bedeuten kann;
R¹, R², R³ und R⁴, die identisch oder voneinander verschieden sind, eine einwertige Gruppe der Formel C_{q}H_{2q}OₛR⁵ₜL, wobei q eine Zahl von 1 bis 8 bedeutet, s und t, die identisch oder voneinander verschieden sind, 0 oder 1 bedeuten, R⁵ eine gegebenenfalls hydroxylierte Alkylengruppe ist und L eine aminierte Gruppe ist, die unter den folgenden Gruppen ausgewählt ist:
-NR"-CH₂-CH₂-N'(R")₂
-N(R")₂,
worin R" Wasserstoff, Phenyl, Benzyl oder eine einwertige gesättigte Kohlenwasserstoffgruppe, beispielsweise eine Alkylgruppe mit 1 bis 20 Kohlenstoffatomen, bedeutet; und
(b) Verbindungen der folgenden Formel (III):
NH-[(CH₂)₃-Si[OSi(CH₃)₃]]₃ (III).

14. Zusammensetzung nach Anspruch 13, **dadurch gekennzeichnet, dass** die Silicone der Formel (II), die mindestens eine Aminogruppe enthalten, ausgewählt sind unter:
(a) Polysiloxanen, die nach CTFA-Nomenklatur als "Amodimethicone" bezeichnet werden und der folgenden Formel (IV) entsprechen: 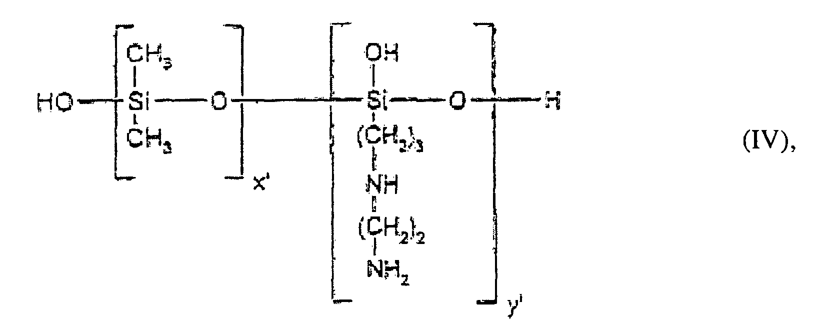 worin x' und y' ganze Zahlen bedeuten, die vom Molekulargewicht abhängen und im Allgemeinen so ausgewählt sind, dass das Molekulargewicht im Bereich von 5 000 bis 20 000 liegt;
(b) Polymeren, die nach CTFA-Nomenklatur als "Trimethylsilylamodimethicon" bezeichnet werden und der folgenden Formel (V) entsprechen: 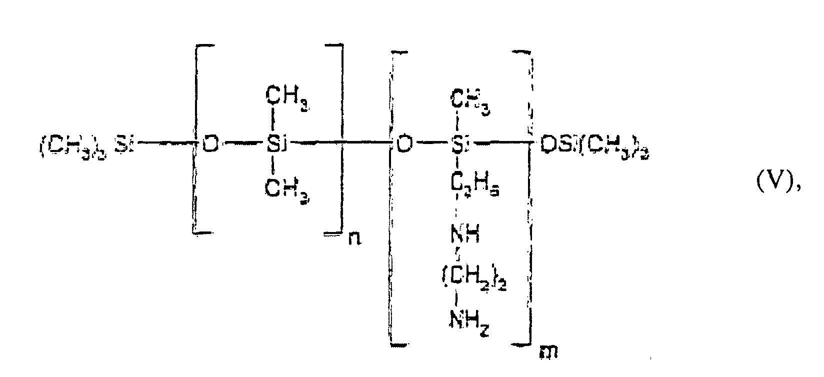
worin n und m die im vorhergehenden Anspruch angegebenen Bedeutungen aufweisen;

15. Zusammensetzung nach einem der Ansprüche 1 bis 14, **dadurch gekennzeichnet, dass** die Silicongummis Polydiorganosiloxane mit einem hohem Molekulargewicht im Bereich von 200 000 bis 2 000 000 sind.

16. Zusammensetzung nach einem der Ansprüche 1 bis 15, **dadurch gekennzeichnet, dass** es sich bei den Polydiorganosiloxanen um ein Polydimethylsiloxan, Polyphenylmethylsiloxan, Poly(diphenylsiloxan-dimethylsiloxan), Poly(dimethylsiloxan-methylvinyisiloxan), Poly(dimethylsiloxanphenylmethylsiloxan) oder Poly(diphenylsiloxan-dimethylsiloxanmethylvinylsiloxan) handelt.

17. Zusammensetzung nach einem der Ansprüche 15 und 16, **dadurch gekennzeichnet, dass** der Silicongummi der folgenden Formel (VI) entspricht: 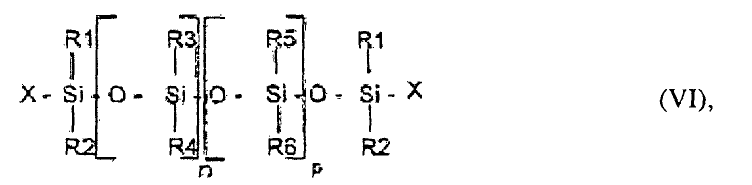 worin bedeuten:
die Gruppen R₁, R₂, R₅ und R₆ gemeinsam oder unabhängig eine Alkylgruppe mit 1 bis 6 Kohlenstoffatomen,
die Gruppen R₃ und R₄ gemeinsam oder unabhängig eine Alkylgruppe mit 1 bis 6 Kohlenstoffatomen oder eine Arylgruppe,
X eine Alkylgruppe mit 1 bis 6 Kohlenstoffatomen, eine Hydroxygruppe oder eine Vinylgruppe,
wobei n und p so ausgewählt sind, das der Silicongummi einen Viskosität über 100 000 mPa·s und vorzugsweise über 500 000 mPa·s aufweist.

18. Zusammensetzung nach einem der Ansprüche 1 bis 17, **dadurch gekennzeichnet, dass** sie ferner mindestens einen Zusatzstoff enthält, der unter den Verdickungsmitteln, Fettsäureestern,
Estern von Fettsäuren und Glycerin, Siliconen, grenzflächenaktiven Stoffen, Parfums, Konservierungsmitteln, Sonnenschutzfiltern, Proteinen, Vitaminen, Polymeren, pflanzlichen Ölen, tierischen Ölen, Mineralölen oder synthetischen
Ölen oder beliebigen herkömmlich in der Kosmetik verwendeten Zusatzstoffen ausgewählt ist.

19. Zusammensetzung nach einem der Ansprüche 1 bis 18, **dadurch gekennzeichnet, dass** das kosmetisch oder dermatologisch akzeptable Medium aus Wasser oder einem Gemisch von Wasser und mindestens einem kosmetisch akzeptablen Lösungsmittel besteht.

20. Zusammensetzung nach Anspruch 19, **dadurch gekennzeichnet, dass** die kosmetisch akzeptablen Lösungsmittel unter Monoalkoholen, Polyalkoholen, Glykolethern, Fettsäureestern und deren Gemischen ausgewählt sind.

21. Zusammensetzung nach einem der Ansprüche 1 bis 20, **dadurch gekennzeichnet, dass** es sich bei den Keratinsubstanzen um menschliches Haar handelt.

22. Zusammensetzung nach einem der Ansprüche 1 bis 21, **dadurch gekennzeichnet, dass** sie als Gel, Milch, Creme, mehr oder weniger dickflüssige Lotion oder Schaum vorliegt.

23. Zusammensetzung nach einem der Ansprüche 1 bis 22, **dadurch gekennzeichnet, dass** es sich um ein Produkt für die Frisurengestaltung handelt.

24. Zusammensetzung nach einem der Ansprüche 1 bis 23, **dadurch gekennzeichnet, dass** es sich um ein Produkt für das Haar handelt, das unter den Haarwaschmitteln und Produkten für das Haar ausgewählt ist, die ausgespült oder nicht ausgespült, vor oder nach einer Haarwäsche, Färbung, Entfärbung, Dauerwelle oder Entkräuselung aufgetragen werden.

25. Zusammensetzung nach einem der Ansprüche 1 bis 24, **dadurch gekennzeichnet, dass** sie in einem Zerstäuber, Pumpflakon oder Aerosolbehälter konfektioniert ist, um ein Spray, einen Lack oder einen Schaum zu erhalten.

26. Zusammensetzung nach einem der Ansprüche 1 bis 25, **dadurch gekennzeichnet, dass** das gepfropfte Siliconpolymer in dem kosmetisch oder dermatologisch akzeptablen Medium gelöst ist oder in Form einer wässrigen Dispersion von Partikeln verwendet wird.

27. Kosmetisches Verfahren zur Behandlung von Keratinsubstanzen und insbesondere menschlichem Haar, **dadurch gekennzeichnet, dass** es darin besteht, auf die Keratinsubstanzen eine Zusammensetzung nach einem der Ansprüche 1 bis 26 aufzutragen und gegebenenfalls mit Wasser zu spülen.

## Claims

1. Cosmetic or dermatological composition intended for treating keratin materials, **characterized in that** it comprises, in a cosmetically or dermatologically acceptable medium, at least:
(a) one silicone polymer containing a polysiloxane skeleton grafted with an organic group not containing silicone, which may be obtained by free radical polymerization between, on the one hand, (i) at least one non-silicone anionic organic monomer containing ethylenic unsaturation and/or one non-silicone hydrophobic organic monomer containing ethylenic unsaturation, and, on the other hand, (ii) a polysiloxane containing in its chain at least one, and preferably more, functional groups capable of reacting with the said ethylenic unsaturations of the said non-silicone monomers; and
(b) at least one silicone chosen from silicones comprising at least one non-quaternized amine function, silicone resins and silicone gums.

2. Composition according to Claim 1, **characterized in that** the anionic organic monomer containing ethylenic unsaturation is chosen, alone or in the form of a monomer mixture, from linear or branched, unsaturated carboxylic acids.

3. Composition according to Claim 2, **characterized in that** the anionic organic monomer containing ethylenic unsaturation is chosen, alone or in the form of a monomer mixture, from acrylic acid, methacrylic acid, maleic acid, maleic anhydride, itaconic acid, fumaric acid and crotonic acid, or alkali metal salts, alkaline-earth metal salts or ammonium salts thereof, or mixtures thereof.

4. Composition according to Claim 1, **characterized in that** the hydrophobic organic monomer containing ethylenic unsaturation is chosen, alone or as a monomer mixture, from acrylic acid esters of alkanol and/or methacrylic acid esters of alkanol, the alkanol preferably being a C₁-C₁₈ alkanol.

5. Composition according to Claim 4, **characterized in that** the hydrophobic organic monomer containing ethylenic unsaturation is chosen, alone or as a monomer mixture, from the group consisting of isooctyl (meth)acrylate, isonyl (meth)acrylate, 2-ethylhexyl (meth)acrylate, lauryl (meth)acrylate, isopentyl (meth)acrylate, n-butyl (meth)acrylate, isobutyl (meth)acrylate, methyl (meth)acrylate, tert-butyl (meth)acrylate, tridecyl (meth)acrylate and stearyl (meth)acrylate.

6. Composition according to any one of Claims 1 to 5, **characterized in that** the grafted silicone polymer comprises, on the main silicone chain, at least one organic group of anionic nature obtained by radical (homo)polymerization of at least one anionic monomer of unsaturated carboxylic acid type, partially or totally neutralized in the form of a salt.

7. Composition according to any one of Claims 1 to 6, **characterized in that** the grafted silicone polymer is chosen from silicone polymers containing in their structure the unit of formula (I) below: 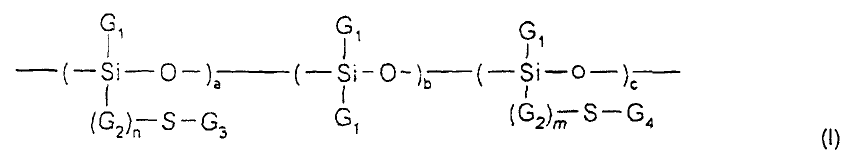 in which the radicals G₁, which may be identical or different, represent hydrogen or a C₁-C₁₀ alkyl radical or alternatively a phenyl radical; the radicals G₂, which may be identical or different, represent a C₁-C₁₀ alkylene group; G₃ represents a polymer residue resulting from the (homo)polymerization of at least one anionic monomer containing ethylenic unsaturation; G₄ represents a polymer residue resulting from the (homo)polymerization of at least one monomer of at least one hydrophobic monomer containing ethylenic unsaturation; m and n are equal to 0 or 1; a is an integer ranging from 0 to 50; b is an integer which may be between 10 and 350, c is an integer ranging from 0 to 50; with the proviso that one of the parameters a and c is other than 0.

8. Composition according to Claim 7, **characterized in that** the unit of formula (I) has at least one of the following characteristics:
- the radicals G₁ denote a C₁-C₁₀ alkyl radical;
- n is non-zero and the radicals G₂ represent a divalent C₁-C₃ radical;
- G₃ represents a polymeric radical resulting from the (homo)polymerization of at least one monomer of the carboxylic acid type containing ethylenic unsaturation;
- G₄ represents a polymeric radical resulting from the (homo)polymerization of at least one monomer of the C₁-C₁₀ alkyl (meth)acrylate type.

9. Composition according to Claim 7 or 8, **characterized in that** the unit of formula (I) simultaneously has the following characteristics:
- the radicals G₁ denote a methyl radical;
- n is non-zero and the radicals G₂ represent a propylene radical;
- G₃ represents a polymeric radical resulting from the (homo)polymerization of at least acrylic acid and/or methacrylic acid;
- G₄ represents a polymeric radical resulting from the (homo)polymerization of at least isobutyl or methyl (meth)acrylate.

10. Composition according to any one of Claims 1 to 9, **characterized in that** the number-average molecular mass of the grafted silicone polymer ranges from 10,000 to 1,000,000, and even more preferably from 10,000 to 100,000.

11. Composition according to any one of Claims 1 to 10, **characterized in that** the grafted silicone polymer(s) is(are) present in concentrations ranging from 0.01 to 20% by weight relative to the total weight of the composition, preferably from 0.1 to 15% by weight and more particularly from 0.5 to 10% by weight.

12. Composition according to any one of Claims 1 to 11, **characterized in that** the silicones containing at least one non-quaternized amine function, the silicone resins and the silicone gums are used in an amount of between 0.01 and 50% by weight relative to the total weight of the composition, preferably between 0.05 and 20% by weight, and, more preferably, this amount is between 0.1 and 10% by weight.

13. Composition according to any one of the preceding claims, **characterized in that** the silicone containing at least one amine function is chosen from:
(a) the silicone polymers corresponding to formula (II) below:
R¹ ₐG¹ ₃₋ₐ-Si(OSiG² ₂)ₙ-(OSiG³ _{b}R² _{2-b})ₘ-O-SiG⁴ ₃₋ₐ'-R³ ₐ' (II)
in which:
G¹, G², G³ and G⁴, which may be identical or different, denote a hydrogen atom or a phenyl, OH, C₁-C₁₈ alkyl, for example methyl, C₂-C₁₈ alkenyl or C₁-C₁₈ alkoxy group,
a and a', which may be identical or different, denote the number 0 or an integer from 1 to 3, in particular 0,
b denotes 0 or 1, and in particular 1,
m and n are numbers such that the sum (n + m) can vary especially from 1 to 2000 and in particular from 50 to 150, it being possible for n to denote a number from 0 to 1999 and in particular from 49 to 149 and for m to denote a number from 1 to 2000 and in particular from 1 to 10;
R¹, R², R³ and R⁴, which may be identical or different, denote a monovalent radical of formula -C_{q}H_{2q}OₛR⁵ₜL in which q is a number from 1 to 8, s and t, which may be identical or different, are equal to 0 or 1, R⁵ denotes an optionally hydroxylated alkylene group and L is an amine group chosen from the groups:
-NR"-CH₂-CH₂-N'(R")₂
-N(R")₂
in which R" can denote hydrogen, phenyl, benzyl or a saturated monovalent hydrocarbon radical, for example an alkyl radical having from 1 to 20 carbon atoms
(b)- compounds of formula (III) below:
NH-[(CH₂)₃-Si[OSi(CH₃)₃]]₃ (III)

14. Composition according to Claim 13, **characterized in that** the silicone containing at least one amine function of formula (II) is chosen from:
(a) the polysiloxanes referred to in the CTFA dictionary as "amodimethicone" and corresponding to formula (IV) below: 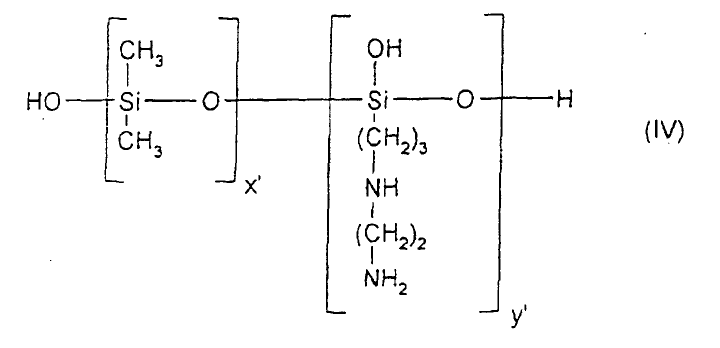 in which x' and y' are integers which are dependent on the molecular weight, generally such that the said molecular weight is between 5000 and 20,000;
(b) the polymers referred to in the CTFA dictionary as "trimethylsilyl amodimethicone", corresponding to formula (V): 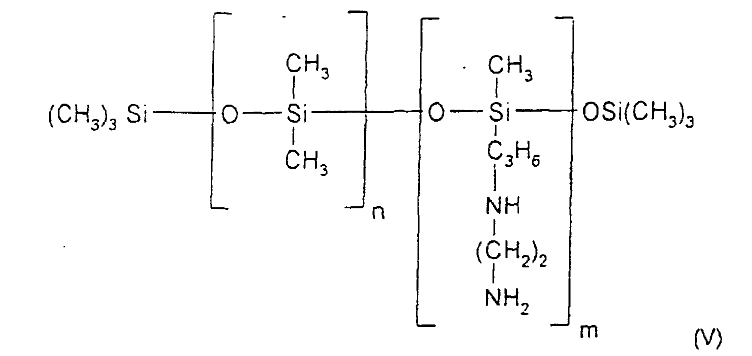
in which n and m have the meanings given in the preceding claim.

15. Composition according to any one of Claims 1 to 14, **characterized in that** the silicone gums are polydiorganosiloxanes with high molecular masses, of between 200,000 and 2,000,000.

16. Composition according to any one of Claims 1 to 15, **characterized in that** the polydiorganosiloxanes are a polydimethylsiloxane, a polyphenylmethylsiloxane, a poly(diphenylsiloxane dimethylsiloxane), a poly(dimethylsiloxane methylvinylsiloxane), a poly(dimethylsiloxane phenylmethylsiloxane), a poly(diphenylsiloxane dimethylsiloxane methylvinylsiloxane).

17. Composition according to either of Claims 15 and 16, **characterized in that** the silicone gum corresponds to formula (VI): 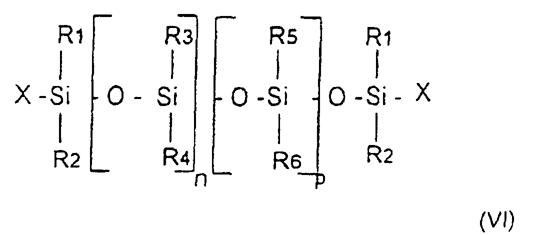 in which:
R₁, R₂, R₅ and R₆ are, together or separately, an alkyl radical having 1 to 6 carbon atoms,
R₃ and R₄ are, together or separately, an alkyl radical having from 1 to 6 carbon atoms, or an aryl radical,
X is an alkyl radical having from 1 to 6 carbon atoms, a hydroxyl radical or a vinyl radical,
n and p being chosen so as to give the silicone gum a viscosity of greater than 100,000 mPa.s, preferably greater than 500,000 mPa.s.

18. Composition according to any one of Claims 1 to 17, **characterized in that** it also contains at least one additive chosen from the group consisting of thickeners, fatty acid esters, fatty acid esters of glycerol, silicones, surfactants, fragrances, preserving agents, sunscreens, proteins, vitamins, polymers, plant, animal, mineral or synthetic oils or any other additive conventionally used in the cosmetics field.

19. Composition according to any one of Claims 1 to 18, **characterized in that** the cosmetically or dermatologically acceptable medium consists of water or a mixture of water and at least one cosmetically acceptable solvent.

20. Composition according to Claim 19, **characterized in that** the cosmetically acceptable solvents are chosen from the group consisting of monoalcohols, polyalcohols, glycol ethers and fatty acid esters, and mixtures thereof.

21. Composition according to any one of Claims 1 to 20, **characterized in that** the keratin material is hair.

22. Composition according to any one of Claims 1 to 21, **characterized in that** it is in the form of a gel, a milk, a cream, a relatively thickened lotion or a mousse.

23. Composition according to any one of Claims 1 to 22, **characterized in that** it is a hairstyling product.

24. Composition according to any one of Claims 1 to 23, **characterized in that** it is a hair product chosen from the group consisting of shampoos, rinse-out or leave-in hair products, to be applied before or after shampooing, dyeing, bleaching, permanent-waving or straightening the hair.

25. Composition according to any one of Claims 1 to 24, **characterized in that** it is packaged in the form of a vaporizer, a pump-dispenser bottle or alternatively in an aerosol container in order to obtain a spray, a lacquer or a mousse.

26. Composition according to any one of Claims 1 to 25, **characterized in that** the grafted silicone polymer is dissolved in the cosmetically or dermatologically acceptable medium or used in the form of an aqueous dispersion of particles.

27. Cosmetic process for treating keratin materials, in particular the hair, **characterized in that** it consists in applying a composition as defined according to any one of Claims 1 to 26 to the said substances and then optionally in rinsing with water.
